# EUROPEAN PATENT SPECIFICATION

(11) **EP 3 705 470 B1**
(45) Date of publication and mention of the grant of the patent: **04.11.2026**
(21) Application number: 20020112.7
(22) Date of filing: 09.03.2020
(51) Int. Cl.: C07C 303/38, C07C 311/48

(54) **NEW PROCESS FOR BISTRIFLUOROMETHANESULFONYLIMIDE SALT**
NEUES VERFAHREN FÜR BISTRIFLUORMETHANSULFONIMIDSALZ
NOUVEAU PROCÉDÉ POUR SEL DE BISTRIFLUOROMÉTHYLSULFONYLIMIDE

(30) Priority: 08.03.2019 CN 201910173903
(43) Date of publication of application: 09.09.2020
(73) Proprietor: Shanghai Rolechem Co., Ltd., Shanghai 201207 (CN)
(72) Inventor: Shen, Fengfeng, Shanghai, 201207 (CN)
(74) Representative: Canzler & Bergmeier Patentanwälte Partnerschaft mbB

(56) References cited:
- ARVAI R ET AL: "A simple access to metallic or onium bistrifluoromethanesulfonimide salts", TETRAHEDRON, ELSEVIER SCIENCE PUBLISHERS, AMSTERDAM, NL, vol. 65, no. 27, 4 July 2009 (2009-07-04), pages 5361 - 5368, XP026149882, ISSN: 0040-4020, [retrieved on 20090503], DOI: 10.1016/J.TET.2009.04.068

## Description

### FIELD OF THE INVENTION

The present disclosure relates to a method for preparing a sulfonylimide salt, and more particularly relates to a method for preparing a bistrifluorosulfonylimide salt.

### BACKGROUND OF THE INVENTION

Bistrifluoromethylsulfonylimide salts (hereinafter abbreviated as MTFSI) have large anion radii, high degree of charge dispersion, very good ionization ability, as well as good chemical stability and thermal stability, and are often used in fields of electrolyte materials, ionic liquids and reaction catalyst, etc.

A method for preparing MTFSI can date back to 1972. In the patent DE2239817, through a four-step reaction, a diperfluoroalkyl sulfonylimide sodium salt was prepared by finally reacting perfluoroalkylsulfonyl fluoride and N-trimethylsilyl perfluorosulfonamide. In 1984, DesMarteau et al. reported preparing diperfluoroalkyl sulfonylimide sodium salt from perfluoroalkylsulfonyl fluoride and N-trimethylsilyl perfluorosulfonamide; then, after acidification by sulfuric acid, a diperfluoroalkyl sulfonylimide can be prepared; by further reaction with salts or oxides, different diperfluoroalkyl sulfonylimide metal salts can be prepared (Foropoulos, J.; DesMarteau, D.D. Inorg. Chem. 1984, 23, 3720-3723).

Another common method for preparing MTFSI is to use anhydrous ammonia, ammonia water or ammonium salt as raw material and use organic amine or nitrogen-containing heterocyclic ring as acid binding agent to react with perfluoroalkyl sulfonyl halide; through perfluorosulfonamide intermediates or further reaction, diperfluoroalkyl sulfonylimide intermediates (or diperfluoroalkyl sulfonylimide ammonium salt intermediates) are prepared, which are then alkalized by basic metal salts or oxides to produce the corresponding diperfluoroalkyl sulfonylimide salts (see DE19533711, CN101456832, CN101983960, US5874616, US20010021790, CN103664712, etc.). Patent CN102153493 uses alkali metal salt directly as the acid binding agent, wherein trifluoromethylsulfonyl halide reacts with anhydrous ammonia or ammonium salt in one step to prepare the corresponding bistrifluoromethylsulfonylimide salt. Patent CN101747244 firstly alkalizes perfluoroalkyl sulfonamide to prepare a metal salt of perfluorosulfonamide to improve its reactivity, which is then reacted with perfluoroalkylsulfonyl fluoride to prepare a diperfluoroalkyl sulfonylimide salt in the presence of carbonate. Other methods for preparing MTFSI include US patent US5072040, in which a perfluoroalkyl sulfonyl halide is reacted with a metal nitride to prepare a diperfluoroalkyl sulfonylimide salt.

Korean patent KR1673535 reported the preparation of N-(trisubstituted silyl) diperfluoroalkyl sulfonylimide through a three-step reaction, and then the organic silicon is removed by a basic metal salt to produce the corresponding diperfluoroalkyl sulfonylimide salt.

In Patent CN105949093, N-benzyl diperfluoroalkyl sulfonylimide is prepared by reacting benzylamine with perfluoroalkyl sulfonyl halide or perfluoroalkylsulfonic anhydride, and then the benzyl group is removed by hydrogenation to prepare diperfluoroalkyl sulfonylimide salt by further alkalization by a basic metal salt.

Roman Arvai et al. reported that N-benzyl diperfluoroalkyl sulfonylimide is prepared by reacting benzylamine with perfluoroalkylsulfonic anhydride, and the N-benzyl diperfluoroalkyl sulfonylimide is then reacted with a basic metal salt after being activated by ethanol to prepare the corresponding diperfluoroalkyl sulfonylimide salt (Tetrahedron 65 (2009) 5361-5368).

The aforementioned preparation methods often require the use of gaseous raw materials, so it is difficult to accurately meter the amount of feed, and the use of ultra-low temperature or high pressure conditions put forward high requirements on the equipment; moreover, the raw materials are expensive or difficult to prepare, the reaction time is long, it is difficult to purify the product, the operations are complicated, environmental pollution is considerable, and there is much room for improvement in the synthesizing process.

In view of the above problems, a new process for a bistrifluoromethylsulfonylimide salt needs to be developed in the art.

### SUMMARY OF THE INVENTION

The present disclosure aims to solve the technical problems that in the existing methods for preparing a bistrifluoromethylsulfonylimide salt, by-products are produced, the operations are complicated, and the amount of three wastes is large, which are adverse to an industrial production. In view of these defects, the present disclosure provides a process for synthesizing a bistrifluoromethylsulfonylimide salt with excellent performance and high purity.

In order to solve the above technical problems, the present disclosure provides a new process for bistrifluorosulfonylimide salt.

As a preferred technical solution, a method for preparing the bistrifluorosulfonylimide salt includes the following steps:
S1: using primary amine and trifluoromethylsulfonic anhydride to prepare the corresponding N-alkyl substituted bistrifluoromethylsulfonylimide;
S2: reacting the N-alkyl substituted bistrifluoromethylsulfonylimide with a salt to obtain a crude product; and
S3: crystallizing and vacuum-drying the crude product to obtain a bistrifluoromethylsulfonylimide salt, wherein the salt is sodium ethoxide, lithium fluoride, or cesium carbonate; wherein the alkyl in the N-alkyl substituted bistri fluoromethylsulfonylimide issaturated aliphatic alkyl or saturated aliphatic alkyl substituted with other elements, and the other elements are one of fluorine, chlorine, bromine, iodine, sulfur, phosphorus, oxygen, and arsenic.

As a preferred technical solution, a molar ratio of the N-alkyl substituted bistrifluoromethylsulfonylimide to the salt is 1: (1~ 3).

As a preferred technical solution, the N-alkyl substituted bistrifluoromethylsulfonylimide has the following characteristic structure: 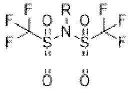 the R is -Cₙ₇Hₙ₈, wherein each of n7 and n8 is a natural number ≥ 1.

As a preferred technical solution, the R is selected from one of saturated aliphatic alkyl, saturated aliphatic alkyl substituted with other elements, and said other elements are one of fluorine, chlorine, bromine, iodine, sulfur, phosphorus, oxygen, and arsenic.

As a preferred technical solution, a solvent is used in the method, the solvent is selected from any one or a combination of more than one of: diethyl carbonate, dimethyl carbonate, propylene carbonate, ethylene carbonate, ethyl methyl carbonate, tetrahydrofuran, methyl tetrahydrofuran, toluene, xylene, chlorobenzene, dichlorobenzene, trichlorobenzene, tetrachlorobenzene, methyl acetate, ethyl acetate, propyl acetate, isopropyl acetate, butyl acetate, acetonitrile, ethylene glycol dimethyl ether, ethylene glycol diisopropyl ether, diethylene glycol dimethyl ether, acetone, methyl isobutyl ketone, diethyl ether, propyl ether, butyl ether, anisole, diphenyl ether, 1,4-dioxane, dichloromethane, trichloromethane, tetrachloromethane, dichloroethane, trichloroethane, tetrachloroethane, tetrachloroethylene and γ-butyrolactone.

As a preferred technical solution, the reaction temperature of step S2 is 0°C to 150°C, and the reaction pressure is 101 KPa to 150 KPa.

The present disclosure has the following advantageous effects:, the N-alkyl substituted bistrifluoromethylsulfonylimide is stable in nature, no corrosive substances are generated during the entire reaction, and there are fewer three wastes, which makes the present disclosure suitable for large-scale production, and enables a high-purity battery-grade bistrifluoromethylsulfonylimide salt to be obtained; therefore, the present disclosure has great implementation value and social and economic benefits.

### DETAILED DESCRIPTION OF THE EMBODIMENT(S) OF THE INVENTION

For the purpose of the following detailed description, it should be understood that various alternative variations and orders of steps may be adopted in the present disclosure, unless expressly specified to the contrary. Furthermore, all numbers indicating quantities of components such as those used in the specification and claims should be construed as being modified in all cases by the term "about", except in any instance of operation, or except in a case where they are otherwise indicated. Accordingly, unless indicated to the contrary, the numerical parameters set forth in the following description and claims are approximations that vary depending on desired performances to be achieved by the present disclosure. At least, it is not intended to limit the applicability of the principle of equivalence to the scope of the claims, and each numerical parameter should be interpreted at least according to the number of reported significant figures and by applying ordinary rounding techniques.

Although the ranges of numerical values and parameters that set forth the broad scope of the present disclosure are approximations, the numerical values listed in specific examples are reported as accurately as possible. Any numerical value, however, inherently contains certain errors necessarily resulting from the standard deviation found in their respective tests and measurements.

In addition, it should be understood that any numerical range described herein is intended to include all sub-ranges contained therein. For example, the range of "1 to 10" is intended to include all sub-ranges between (and including) the minimum value 1 and the maximum value 10, that is, having a minimum value equal to or greater than 1 and a maximum value equal to or less than 10.

Drugs or components not mentioned in the present disclosure are all commercially available.

In order to solve the above technical problems, the present disclosure provides a new process for a bistrifluorosulfonylimide salt.

In a specific embodiment, the method for preparing a bistrifluorosulfonylimide salt includes the following steps:
S1: using primary amine and trifluoromethylsulfonic anhydride to prepare the corresponding N-alkyl substituted bistrifluoromethylsulfonylimide;
S2: reacting the N-alkyl substituted bistrifluoromethylsulfonylimide with a salt to obtain a crude product; and
S3: crystallizing and vacuum-drying the crude product to obtain a bistrifluoromethylsulfonylimide salt wherein the salt is sodium ethoxide, lithium fluoride, or cesium carbonate; wherein the alkyl in the N-alkyl substituted bistri fluoromethylsulfonylimide issaturated aliphatic alkyl or saturated aliphatic alkyl substituted with other elements, and the other elements are one of fluorine, chlorine, bromine, iodine, sulfur, phosphorus, oxygen, and arsenic.

The generic reaction equation of the present disclosure is as follows:

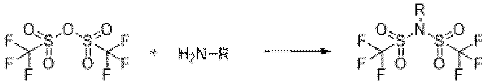

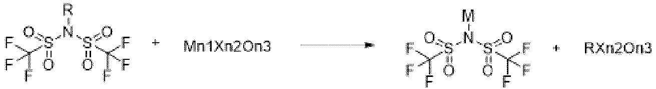

In a specific embodiment, a molar ratio of the N-alkyl substituted bistrifluoromethylsulfonylimide to the salt is 1: (1~ 3).

In a preferred embodiment, the molar ratio of the N-alkyl substituted bistrifluoromethylsulfonylimide to the salt is 1: 1.2.

In a specific embodiment, the N-alkyl substituted bistrifluoromethylsulfonylimide has the following characteristic structure: 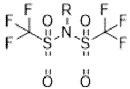 the R is -Cₙ₇Hₙ₈, wherein each of n7 and n8 is a natural number ≥ 1.

The applicant has found that the use of the N-alkyl substituted bistrifluoromethylsulfonylimide can ensure that the substances are stable during the reaction and no corrosive substances are generated during the reaction.

This is because the use of the N-alkyl substituted bistrifluoromethylsulfonylimide enables the system to be neutral during the reaction, and avoids the generation of corrosive substances.

In a preferred embodiment, the R is selected from one of saturated aliphatic alkyl, saturated aliphatic alkyl substituted with other elements, and said other elements are one of fluorine, chlorine, bromine, iodine, sulfur, phosphorus, oxygen, and arsenic.

By selecting a suitable salt and N-alkyl substituted bistrifluoromethylsulfonylimide, the present application can reduce the generation of by-products, overcome the technical defects of a large amount of three wastes, and simplify the production steps, thereby making this technology suitable for industrial production.

In a specific embodiment, the solvent is selected from any one or a combination of more than one of: diethyl carbonate, dimethyl carbonate, propylene carbonate, ethylene carbonate, ethyl methyl carbonate, tetrahydrofuran, methyl tetrahydrofuran, toluene, xylene, chlorobenzene, dichlorobenzene, trichlorobenzene, tetrachlorobenzene, methyl acetate, ethyl acetate, propyl acetate, isopropyl acetate, butyl acetate, acetonitrile, ethylene glycol dimethyl ether, ethylene glycol diisopropyl ether, diethylene glycol dimethyl ether, acetone, methyl isobutyl ketone, diethyl ether, propyl ether, butyl ether, anisole, diphenyl ether, 1,4-dioxane, dichloromethane, trichloromethane, tetrachloromethane, dichloroethane, trichloroethane, tetrachloroethane, tetrachloroethylene and γ-butyrolactone.

A solvent is a liquid that can dissolve gas, solid, and liquid into a homogeneous mixture. Gases and solids are conventionally called solutes, and liquids are called solvents. For a solution consisting of two liquids, the component with a higher content is usually called the solvent, and the component with a lower content is called the solute. The solvent is divided into two major categories of inorganic solvents and organic solvents. Water is the most widely used inorganic solvent, and alcohol, gasoline, chloroform, and acetone are commonly used organic solvents. Therefore, there is no distinction of optimal and worst, and a choice is made according to actual needs.

In a specific embodiment, the reaction temperature of step S2 is 0°C to 150°C, and the reaction pressure is 101 KPa to 150 KPa.

In a preferred embodiment, the reaction temperature of step S2 is 60°C, and the reaction pressure is 125 KPa.

The present disclosure will be further described below by way of example, but it is not intended to limit the present disclosure to the scope of the described example. For the experimental methods without specific conditions specified in the following example, choices are made according to conventional methods and conditions, or according to product specifications. The reaction pressures in Examples 1 to 4 are 125 KPa.

### Examples

### Example 1

Example 1 provides a method for preparing a bistrifluorosulfonylimide salt, which includes the following steps:
under nitrogen protection, adding 100 ml of tetrahydrofuran solution of ethylamine (concentration: 2 moles/liter) and 44.52 g of triethylamine to 620.8 g of dry dichloromethane; after cooling to -78°C, starting adding 124.14 g of trifluoromethylsulfonic anhydride dropwise for about 2 hours; after the dropwise addition is completed, maintaining the temperature for 2 hours, and then slowly raising the temperature for about 1 hour to reach room temperature; continuing the reaction at the room temperature for 1 hour; adding 300 g of water, then extracting with dichloromethane three times, combining the organic phases, removing the solvent, and then vacuum-distilling to obtain 52.06 g of N-ethyl-bistrifluoromethylsulfonylimide.

1HNMR (400MHz, CDCl3) δ (ppm): 0.98 (t, 3H), 3.87 (q, 2H), 19FNMR (300MHz, CDCl3) δ (ppm): -72.36.

adding 13.75 g of sodium ethoxide and 52.06 g of prepared N-ethyl-bistrifluoromethylsulfonylimide to 260.3 g of dimethyl carbonate, raising the temperature to 60°C for reaction for 12 hours, and lowering the temperature to the room temperature; then performing filtration and vacuum-concentration to reach a thick state; adding 200 g of toluene dropwise, stirring at 20°C for 2 hours, followed by filtering and washing with toluene; and vacuum-drying the filtered cake at 90°C to obtain 47.57 g of sodium bistrifluoromethylsulfonylimide, with the yield being 93.2%, moisture being less than 100 ppm, and acid value being less than 100 ppm.

19FNMR (300MHz, CD3CN) δ (ppm): -79.26.

### Example 2

Example 2 provides a method for preparing a bistrifluorosulfonylimide salt, which includes the following steps:
under nitrogen protection, adding 14.63 g of butylamine and 48.57 g of triethylamine to 620.69 g of dry dichloromethane; after cooling to -78°C, starting adding 124.14 g of trifluoromethylsulfonic anhydride dropwise for about 2 hours; after the dropwise addition is completed, maintaining the temperature for 1 hour, and then slowly raising the temperature for about 1 hour to reach room temperature; continuing the reaction at the room temperature for 1 hour; adding 300 g of water, then extracting with dichloromethane three times, combining the organic phases, vacuum-removing the solvent to obtain a concentrated solution, and vacuum-distilling to obtain 51.26 g of N-butyl-bistrifluoromethylsulfonylimide.

1HNMR (400MHz, CDCl3) δ (ppm): 0.95 (t, 3H), 1.30-1.42 (m, 2H), 1.73-1.86 (m, 2H), 3.92 (t, 2H), 19FNMR (300MHz, CDCl3) δ (ppm): -71.75.

adding 5.91 g of anhydrous lithium fluoride and 51.26 g of N-butyl-bistrifluoromethylsulfonylimide to 250 g of isopropyl acetate, incurring a reflux reaction for 10 hours, and lowering the temperature to the room temperature, followed by filtration and vacuum concentration of filtrate to reach a thick state; adding 200 g of dichloromethane dropwise, stirring at 20°C for 2 hours, followed by filtering and washing with dichloromethane; and vacuum-drying the filtered cake at 80°C to obtain 39.57 g of lithium bistrifluoromethylsulfonylimide, with the yield being 90.7%, moisture being less than 100 ppm, and acid value being less than 100 ppm.

19FNMR (300MHz, CD3CN) δ (ppm): -79.23.

### Example 3

Example 3 provides a method for preparing a bistrifluorosulfonylimide salt, which includes the following steps:
under nitrogen protection, adding 13.50 g of methylamine hydrochloride to 310.3 g of dichloromethane; after cooling to -78°C, adding 44.52 g of triethylamine dropwise, and then slowly adding 62.06 g of trifluoromethylsulfonic anhydride dropwise for about 2 hours; after the dropwise addition is completed, maintaining the temperature for 1 hour, and then slowly raising the temperature for about 1 hour to reach room temperature; continuing the reaction at the room temperature for 1 hour; adding 150 g of water, then extracting with dichloromethane three times, combining the organic phases, vacuum-removing the solvent, and vacuum-distilling to obtain 28.05 g of N-methyl-bistrifluoromethylsulfonamide.

1HNMR (400MHz, CDCl3) δ (ppm): 3.82 (s, 3H), 19FNMR (300MHz, CDCl3) δ (ppm): -73.91.

under nitrogen protection, adding 28.05 g of N-methyl-bistrifluoromethylsulfonamide obtained above and 20.88 g of triethylamine to 291.16 g of dry dichloromethane; after cooling to -78°C, starting adding 58.23 g of trifluoromethylsulfonic anhydride dropwise for about 2 hours; after the dropwise addition is completed, maintaining the temperature for 1 hour, and then slowly raising the temperature for about 1 hour to reach room temperature; continuing the reaction at the room temperature for 1 hour; adding 150 g of water, then extracting with dichloromethane three times, combining the organic phases, vacuum-removing the solvent to obtain a concentrated solution, and vacuum-distilling to obtain 41.12 g of N-methyl-bistrifluoromethylsulfonylimide.

under nitrogen protection, adding 41.12 g of N-methyl-bistrifluoromethylsulfonylimide prepared above and 113.22 g of anhydrous cesium carbonate to 400 g of acetonitrile, raising the temperature and incurring a reflux reaction for 15 hours, and lowering the temperature to the room temperature, followed by filtration and vacuum concentration to reach a thick state; adding 200 g of dichloromethane dropwise, stirring at 20°C for 2 hours, followed by filtering and washing with dichloromethane; and vacuum-drying the filtered cake at 70°C to obtain 52.70 g of cesium bistrifluoromethylsulfonylimide, with the yield being 91.8%, moisture being less than 100 ppm, and acid value being less than 100 ppm.

19FNMR (300MHz, CD3CN) δ (ppm): -79.31.

### Example 4

Example 4 provides a method for preparing a bistrifluorosulfonylimide salt, which includes the following steps:
under nitrogen protection, adding 17.13 g of benzylamine and 51.70 g of diisopropylethylamine to 705.35 g of dry dichloromethane; after cooling to -78°C, starting adding 141.07 g of trifluoromethylsulfonic anhydride dropwise for about 2 hours; after the dropwise addition is completed, maintaining the temperature for 1 hour, and then slowly raising the temperature for about 1 hour to reach room temperature; continuing the reaction at the room temperature for 1 hour; adding 300 g of water, then extracting with dichloromethane three times, combining the organic phases, drying with anhydrous magnesium sulfate, filtering, vacuum-removing the solvent to obtain a concentrated solution which is refluxed with n-hexane, and hot-extracting 5 times; collecting and combining the n-hexane phases, and vacuum-removing the solvent to obtain 62.15 g of N-benzyl-bisfluorosulfonylimide.

1HNMR (400MHz, CDCl3) δ (ppm): 5.10 (s, 2H), 7.36-7.43 (m, 3H), 7.47-7.52 (m, 2H), 19FNMR (300MHz, CDCl3) δ (ppm): -72.64.

adding 62.15 g of N-benzyl-bisfluorosulfonylimide prepared above and 11.27 g of anhydrous potassium hydroxide to 311 g of ethylene glycol dimethyl ether, reacting at 70°C for 10 hours, and lowering the temperature to the room temperature, followed by filtration and vacuum concentration to reach a thick state; adding 200 g of toluene dropwise, stirring at 20°C for 2 hours, followed by filtering and washing with toluene; and vacuum-drying the filtered cake at 80°C to obtain 45.42 g of potassium bistrifluoromethylsulfonylimide, with the yield being 85.2%, moisture being less than 100 ppm, and acid value being less than 100 ppm.

19FNMR (300MHz, CD3CN) δ (ppm): -79.27

The foregoing examples are merely illustrative and are used to explain some features of the method according to the present disclosure. The appended claims are intended to claim the widest possible scope that can be conceived, and the examples presented herein are merely illustrative of selected embodiments based on combinations of all possible examples. Accordingly, it is the applicant's intention that the appended claims not be limited by the selection of examples illustrating the features of the present disclosure. Some numerical ranges used in the claims also include subranges contained therein, and variations in these ranges should also be interpreted, where possible, as being covered by the appended claims.

## Claims

1. A method for preparing a bistrifluoromethylsulfonylimide salt, comprising the following steps:
S1: using primary amine and trifluoromethylsulfonic anhydride to prepare the corresponding N-alkyl substituted bistrifluoromethylsulfonylimide;
S2: reacting the N-alkyl substituted bistrifluoromethylsulfonylimide with a salt to obtain a crude product; and
S3: crystallizing and vacuum-drying the crude product to obtain a bistrifluoromethylsulfonylimide salt,
wherein the salt is sodium ethoxide, lithium fluoride, or cesium carbonate;
wherein the alkyl in the N-alkyl substituted bistrifluoromethylsulfonylimide is saturated aliphatic alkyl or saturated aliphatic alkyl substituted with other elements, and the other elements are one of fluorine, chlorine, bromine, iodine, sulfur, phosphorus, oxygen, and arsenic.

2. The preparing method according to claim 1, wherein a molar ratio of the N-alkyl substituted bistrifluoromethylsulfonylimide to the salt is 1:(1~3).

3. The preparing method according to claim 1, wherein a solvent is used in the method and the solvent is selected from any one or a combination of more than one of: diethyl carbonate, dimethyl carbonate, propylene carbonate, ethylene carbonate, ethyl methyl carbonate, tetrahydrofuran, methyl tetrahydrofuran, toluene, xylene, chlorobenzene, dichlorobenzene, trichlorobenzene, tetrachlorobenzene, methyl acetate, ethyl acetate, propyl acetate, isopropyl acetate, butyl acetate, acetonitrile, ethylene glycol dimethyl ether, diethylene glycol dimethyl ether, acetone, methyl isobutyl ketone, diethyl ether, propyl ether, butyl ether, anisole, diphenyl ether, 1,4-dioxane, dichloromethane, trichloromethane, tetrachloromethane, dichloroethane, trichloroethane, tetrachloroethane, tetrachloroethylene and γ-butyrolactone.

4. The preparing method according to claim 1, wherein the reaction temperature of step S2 is 0°C to 150°C, and the reaction pressure is 101KPa to150 KPa.

## Patentansprüche

1. Verfahren zum Herstellen eines Bistrifluormethylsulfonylimid-Salzes, umfassend die folgenden Schritte:
S1: Verwenden von primärem Amin und Trifluormethylsulfonsäureanhydrid, um das entsprechende N-alkylsubstituierte Bistrifluormethylsulfonylimid herzustellen;
S2: Reagieren des N-alkylsubstituierten Bistrifluormethylsulfonylimids mit einem Salz, um ein Rohprodukt zu erhalten; und
S3: Kristallisieren und Vakuumtrocknen des Rohprodukts, um ein Bistrifluormethylsulfonylimid-Salz zu erhalten,
wobei das Salz Natriumethoxid, Lithiumfluorid oder Cäsium-Karbonat ist;
wobei das Alkyl in dem N-alkylsubstituierten Bistrifluormethylsulfonylimid gesättigtes aliphatisches Alkyl oder gesättigtes aliphatisches Alkyl, substituiert mit anderen Elementen, ist und die anderen Elemente eines von Fluor, Chlor, Brom, Jod, Schwefel, Phosphor, Sauerstoff und Arsen sind.

2. Herstellungsverfahren nach Anspruch 1, wobei ein Molverhältnis des N-alkylsubstituierten Bistrifluormethylsulfonylimids zu dem Salz 1:(1~3) ist.

3. Herstellungsverfahren nach Anspruch 1, wobei ein Lösungsmittel in dem Verfahren verwendet wird und das Lösungsmittel ausgewählt ist aus einem oder einer Kombination von mehr als einem von: Diethylkarbonat, Dimethylkarbonat, Propylenkarbonat, Ethylenkarbonat, Ethylmethylkarbonat, Tetrahydrofuran, Methyltetrahydrofuran, Toluol, Xylol, Chlorbenzol, Dichlorbenzol, Trichlorbenzol, Tetrachlorbenzol, Methylazetat, Ethylazetat, Propylazetat, Isopropylazetat, Butylazetat, Azetonitril, Ethylenglykoldimethylether, Diethylenglykoldimethylether, Azeton, Methylisobutylketon, Diethylether, Propylether, Butylether, Anisol, Diphenylether, 1,4-Dioxan, Dichlormethan, Trichlormethan, Tetrachlormethan, Dichlorethan,Trichlorethan, Tetrachlorethan, Tetrachlorethylen und γ-Butyrolacton.

4. Herstellungsverfahren nach Anspruch 1, wobei die Reaktionstemperatur von Schritt S2 0°C bis 150°C ist und der Reaktionsdruck 101 KPa bis 150 KPa ist.

## Revendications

1. Procédé pour préparer un sel de bistrifluorométhylsulfonylimide, comprenant les étapes suivantes :
S1 : utiliser une amine primaire et un anhydride trifluorométhylsulfonique pour préparer le bistrifluorométhylsulfonylimide substitué par un N-alkyle correspondant ;
S2 : faire réagir le bistrifluorométhylsulfonylimide substitué par un N-alkyle avec un sel pour obtenir un produit brut ; et
S3 : cristalliser et sécher sous vide le produit brut pour obtenir un sel de bistrifluorométhylsulfonylimide,
dans lequel le sel est l'éthoxyde de sodium, le fluorure de lithium ou le carbonate de césium ;
dans lequel l'alkyle dans le bistrifluorométhylsulfonylimide substitué par un N-alkyle est un alkyle aliphatique saturé ou un alkyle aliphatique saturé substitué par d'autres éléments, et les autres éléments sont l'un parmi le fluor, le chlore, le brome, l'iode, le soufre, le phosphore, l'oxygène et l'arsenic.

2. Procédé de préparation selon la revendication 1, dans lequel un rapport molaire du bistrifluorométhylsulfonylimide substitué par un N-alkyle au sel est de 1:(1~3).

3. Procédé de préparation selon la revendication 1, dans lequel un solvant est utilisé dans le procédé et le solvant est choisi parmi l'un quelconque ou une combinaison de plus d'un parmi : le carbonate de diéthyle, le carbonate de diméthyle, le carbonate de propylène, le carbonate d'éthylène, le carbonate d'éthyle et de méthyle, le tétrahydrofurane, le méthyltétrahydrofurane, le toluène, le xylène, le chlorobenzène, le dichlorobenzène, le trichlorobenzène, le tétrachlorobenzène, l'acétate de méthyle, l'acétate d'éthyle, l'acétate de propyle, l'acétate d'isopropyle, l'acétate de butyle, l'acétonitrile, l'éther diméthylique d'éthylène glycol, l'éther diméthylique de diéthylène glycol, l'acétone, la méthylisobutylcétone, l'éther diéthylique, l'éther propylique, l'éther butylique, l'anisole, l'éther diphénylique, le 1,4-dioxane, le dichlorométhane, le tri-chlorométhane, le tétrachlorométhane, le dichloroéthane, le trichloroéthane, le tétrachloroéthane, le tétrachloroéthylène et la γ-butyrolactone.

4. Procédé de préparation selon la revendication 1, dans lequel la température de réaction de l'étape S2 est de 0 °C à 150 °C, et la pression de réaction est de 101 KPa à 150 KPa.
